(19) Europäisches Patentamt
European Patent Office
Office européen des brevets

(11) Publication number: 0 135 767
A1

(12) # EUROPEAN PATENT APPLICATION

(21) Application number: 84109567.2

(22) Date of filing: 10.08.84

(51) Int. Cl.⁴: C 07 D 223/16, A 61 K 31/55, C 07 D 401/06, C 07 D 401/04, C 07 D 405/06, C 07 D 405/04, C 07 D 409/06, C 07 D 409/04, C 07 D 403/04, C 07 D 413/04

(30) Priority: 12.08.83 US 522948

(43) Date of publication of application: 03.04.85
Bulletin 85/14

(84) Designated Contracting States: IT

(71) Applicant: SCHERING CORPORATION, 2000 Galloping Hill Road, Kenilworth, New Jersey 07033 (US)

(72) Inventor: Berger, Joel Gilbert, 54 Park Avenue, Verona, NJ 07044 (US)
Inventor: Chang, Wei Kong, 63 West Cedar Street, Livingston, NJ 07039 (US)
Inventor: Gold, Elijah Herman, 10 Roosevelt Avenue, West Orange, NJ 07052 (US)
Inventor: Elliott, Arthur John, 62 Seven Lakes Drive, Sloatsburg, NY 10974 (US)

(74) Representative: Antony, Fritz, Dr. et al, c/o Scherico Ltd. P.O. Box 601 Töpferstrasse 5, CH-6002 Lucerne (CH)

(54) **Benzazepine derivatives, pharmaceutical compositions containing them and processes for the preparation of such compounds and compositions.**

(57) Substituted 8-amino-1-phenyl-2,3,4,5-tetrahydro-1H-3-benzazepines useful in the treatment of mental disorders such as schizophrenia and depression, and having activities of prolonged duration, are disclosed. Methods for preparing these compounds and methods for their use are also described.

EP 0 135 767 A1

BENZAZEPINE DERIVATIVES, PHARMACEUTICAL COMPOSITIONS CONTAINING THEM AND PROCESSES FOR THE PREPARATION OF SUCH COMPOUNDS AND COMPOSITIONS

This invention relates to benzazepine derivatives useful in the treatment of mental disorders.

British patent specification 1,268,243 generically discloses a class of benzazepines which may contain an 8-amino and a 1-phenyl substituent. The specification does not specifically describe any such compounds, nor does it disclose how the 8-amino-1-phenyl substituted benzazepines may be prepared. The disclosed compounds are described as having analgesic, antihistaminic, anticholinergic and narcotic-antagonist properties.

The present invention provides certain substituted 8-amino-1-phenyl-2,3,4,5-tetrahydro-1H-3-benzazepines which possess properties useful for the treatment of mental disorders such as schizophrenia and depression. These compounds have the structural formula I

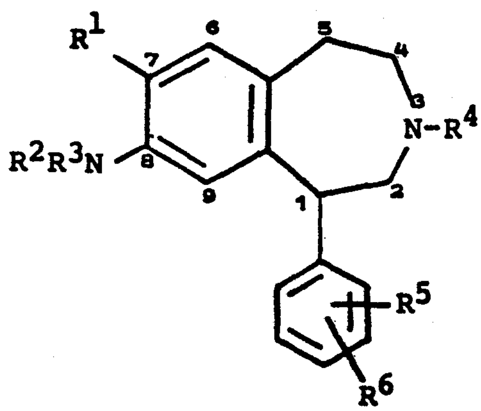

I

wherein $R^1$ is a hydrogen or halogen atom or an alkyl, $S(O)_n$.alkyl or alkoxy group having from 1 to 6 carbon atoms, wherein n is 0, 1 or 2 or a trifluoromethyl group;

$R^2$ is a hydrogen atom, a group $R^a$ [wherein $R^a$ is an alkyl group having from 1 to 6 carbon atoms, a group $-(CH_2)_m$-Aryl, (wherein m is 0 or an integer of 1 to 3 and Aryl is a group

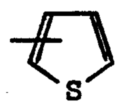, 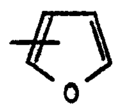, 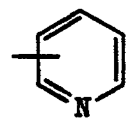, 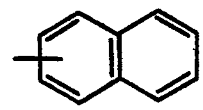 or 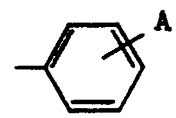

wherein A is a hydrogen or halogen atom, an alkyl, $S(O)_n$.alkyl or alkoxy group having from 1 to 6 carbon atoms, wherein n is 0, 1 or 2, or a phenyl, hydroxy or trifluoromethyl group)], or a group $C(O)XR^a$ [wherein X is a bond, O, or $NR^b$ (wherein $R^b$ is a hydrogen atom or an alkyl group having from 1 to 6 carbon atoms), $R^a$ is as defined above and $XR^a$ may be $NH_2$],

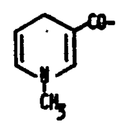,

$SO_2R^a$ or $CONHSO_2R^a$ (wherein $R^a$ is as defined above);

$R^3$ is a hydrogen atom or a formyl group or, when $R^2$ is an alkyl group having from 1 to 6 carbon atoms, $R^3$ can also be an alkyl group having from 1 to 6 carbon atoms or a group $R^a$ as defined above;

or $R^2$ and $R^3$ together with the nitrogen atom to which they are attached form a group

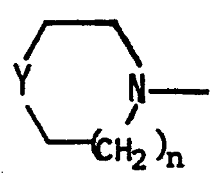

wherein Y is O, $CH_2$ or $NR^b$ [wherein $R^b$ is as defined above], and _n_ is 0, 1 or 2 provided that _n_ is not 0 when Y is O or $NR^b$;

$R^4$ is a hydrogen atom or an alkyl group having from 1 to 6 carbon atoms;

each of $R^5$ and $R^6$, which may be the same or different, is a hydrogen or halogen atom or an alkyl or $S(O)_n$.alkyl group having from 1 to 6 carbon atoms, a trifluoromethyl group or a group $OR^b$ [wherein $R^b$ is as defined above]; or $R^5$ and $R^6$ together may form a group $-OCH_2O-$ substituting adjacent positions in the fused benzene ring;

and the pharmaceutically acceptable salts thereof.

Halogen atoms may be fluorine, chlorine, bromine or iodine, but chlorine and bromine are preferred. The alkyl and alkoxy groups preferably have up to 4 carbon atoms and may be straight or branched.

Preferred definitions for the above described substituents are as follows:

$R^1$ is a halogen atom or a methyl or trifluoromethyl group;

$R^2$ is a hydrogen atom or a methyl or $SO_2R^a$ group (wherein $R^a$ is as defined above but is preferably an alkyl group having from 1 to 4 carbon atoms);

$R^3$ is a hydrogen atom;

$R^4$ is a methyl group;

$R^5$ is a hydrogen or halogen atom or a methyl group or a group $OR^b$ [wherein $R^b$ is as defined above]; and

$R^6$ is a hydrogen atom.

Compounds of the formula I exist as racemates and as *R* and *S* enantiomers; all such forms are comprised by the present invention. The *R* enantiomers are normally preferred on account of their very favorable pharmacological properties. Two particularly preferred compounds of the invention are (*R*)-8-amino-7-chloro-3-methyl-1-phenyl-2,3,4,5-tetrahydro-1*H*-3-benzazepine and (*R*)-8-methanesulfonamido-7-chloro-3-methyl-1-phenyl-2,3,4,5-tetrahydro-1*H*-3-benzazepine.

Compounds of the formula I are basic by virtue of the nitrogen atom in the azepine ring and therefore form acid addition salts with organic and inorganic acids. Suitable acids for forming pharmaceutically acceptable salts include hydrochloric, sulfuric, phosphoric, acetic, citric, oxalic, malonic, salicylic, malic, fumaric, succinic, ascorbic, maleic and methanesulfonic acids. Compounds of the formula I that contain a sulfonamide group, i.e. those wherein $R^2$ is a group $CONHSO_2R^a$ (wherein $R^a$ is as defined above), are also acidic and form salts with strong bases such as alkali metals, in particular with sodium and potassium.

The compounds of formula I and their salts can exist in unsolvated as well as solvated forms, including hydrated forms. In general, the solvated forms with pharmaceutically acceptable solvents such as water, ethanol and the like are equivalent to the unsolvated forms for purposes of the invention.

The invention further provides pharmaceutical compositions useful for treating mental disorders such as psychoses and/or depression in a mammal which comprise as active ingredient a compound having structural formula I or a pharmaceutically acceptable salt thereof in admixture or association with a pharmaceutically acceptable carrier.

Thus the compounds of the formula I and their pharmaceutically acceptable acid addition salts can be used in the treatment of psychoses or depression in a mammal, in particular by administering an effective amount of the above-defined pharmaceutical composition to said mammal.

The compounds of the invention having structural formula I wherein $R^2=R^3=H$ may be prepared from certain correspondingly substituted 8-acyloxy compounds by methods known in the art; and may then (where necessary) be readily converted into other desired compounds of the invention wherein $R^2$ and $R^3$ may be other than hydrogen.

In the following discussion, the 8-substituted benzazepine moiety will be represented by the symbolic formula I' for reasons of convenience:

(substituent) –Ⓑ I',

wherein the symbol –Ⓑ represents the partial structure

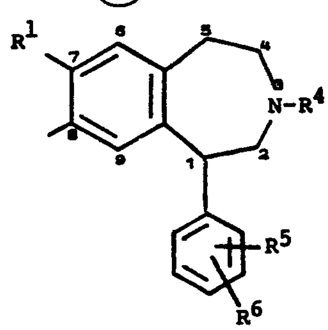

and has the indicated substituent bonded at the 8-position.

According to the invention, we provide a process for the preparation of a compound of the formula I defined above or of a pharmaceutically acceptable salt thereof, which comprises, as main step, hydrolysing an amide of the formula $HO.C(CH_3)_2.CO.NH$–Ⓑ to an 8-amino-benzazepine of the formula $H_2N$–Ⓑ

wherein (B) is a benzazepine-8-yl group of the formula

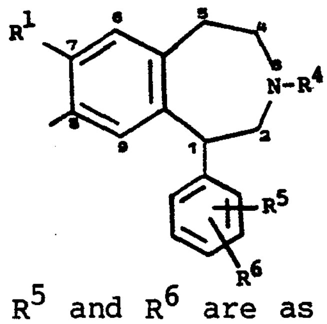

in which $R^1$, $R^4$, $R^5$ and $R^6$ are as defined herein, with the proviso that any hydroxy group in $R^5$ and/or $R^6$ can be protected,
whereafter the resulting 8-amino-benzazepine of the formula

$H_2N$-(B)

can be subjected, as desired or necessary, to one or more of the following finishing steps in any desired order:
(a) Elimination of a 7-halogen atom other than fluorine by catalytic reduction;
(b) Introduction of a 7-alkyl group by oxidative condensation of a 7-unsubstituted-8-$NH_2$-benzazepine with an arylalkylthio ether, followed by reductive cleavage of the resulting 7-arylthioalkyl-benzazepine;
(c) Acylation to a carboxamide $R^aXCO.NH$-(B) or to a sulfonamide $R^aSO_2NH$-(B), wherein X and $R^a$ are as defined above;
(d) Reaction with carbonyldiimidazole followed by the *in situ* reaction of the resulting urea with a primary or secondary alcohol $R^aOH$ or with a primary or secondary amine $R^aNH_2$ or with ammonia, where $R^a$ is as defined above;
(e) Reduction of an acylamide derivative $R^aCONH$-(B) or of a urethane $R^aO(CO)$-NH-(B), wherein $R^a$ and (B) are as defined above, from step (c) to a secondary amine $R^xNH$-(B) or $CH_3NH$-(B) respectively, where $R^x$ is a group as defined above for $R^a$, except that *m* is not 0;

(f) Alkylation of an amide $R^aSO_2NH$-(B) or $R^aCONH$-(B) from step (c) to yield a disubstituted amide $R^aCO.NR^x$-(B) or $R^aSO_2.NR^x$-(B), where $R^a$ and $R^x$ are as defined above;
(g) Acylation of a secondary amine $R^xNH$-(B) from step (e) to a carboxamide $R^aX.CO.NR^x$-(B) or to a sulfonamide $R^aSO_2NR^x$-(B), wherein X, $R^x$ and $R^a$ are as defined above;
(h) Reduction of a carboxamide $R^aCON.R^x$-(B) or $R^aO.CO.NR^x$-(B) from step (f) or (g) to a tertiary amine $R^xR^xN$-(B) or $CH_3N.R^x$-(B) or of a sulfonamide $R^aSO_2.NR^x$-(B) from step (g) to a secondary amine $R^xNH$-(B), wherein $R^a$ and $R^x$ are as defined above;
(i) Hydrolysis of an amide $R^aCO.NR^x$-(B) from step (f) to a secondary amine $R^x.NH$-(B), wherein $R^a$ and $R^x$ are as defined above;
(j) Reductive alkylation of the primary amine $H_2N$-(B) from the main step, step (a) or step (b), or of a secondary amine $R^xNH$-(B) from step (e) or step (h) with an aldehyde or ketone and a reducing agent to yield a secondary amine $R^xNH$-(B) or tertiary amine $R^xR^xN$-(B);
(k) Acylation of the primary amine $H_2N$-(B) from the main step, step (a) or step (b) with nicotinic acid or with a reactive derivative thereof to yield an amide of the formula

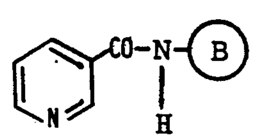

followed by N-methylation in the pyridine ring and 1,4-reduction of the resulting N-methyl-pyridinium salt;
(l) Acylation of an amine $H_2N$-(B) with an aromatic carboxylic acid, conversion of the resulting amide via the chloro-imidate into an aryl-imidate by reaction with a phenol of the formula

Aryl-OH,

thermal rearrangement of the aryl-imidate to an aroyl-diarylamine, and hydrolysis of this aroyl-diarylamine to an amine of the formula

Aryl-NH-Ⓑ,

wherein Aryl is as defined above;

(m) Condensation of an N-acetyl-amine of the formula $CH_3CONH$-Ⓑ from step (c) with an aryl bromide Aryl.Br in the presence of copper powder to an acetyl-diarylamine, and hydrolysis of this acetyl-diarylamine to an amine of the formula Aryl.NH-Ⓑ wherein the group Aryl is as defined above;

(n) Acylation of the amino compound $H_2N$-Ⓑ with an acylating derivative of the acid $L(CH_2)_nCH_2YCH_2CO_2H$ to produce an amide having the formula

$L(CH_2)_nCH_2YCH_2CONH$-Ⓑ V

wherein L is a leaving group such as halogen and Y and n are as defined above, which is then cyclised to yield a cyclic amide which in turn is reduced to an amine of the formula

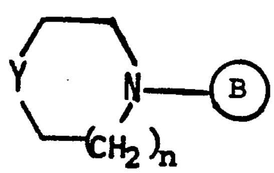

VI

wherein Y and n are as defined above;

(o) Removal of any group protecting a hydroxy group in $R^5$ and/or $R^6$;

(p) Isolation of the compound of formula I as the free base or as a pharmaceutically acceptable salt, as the racemate or as the resolved R or S enantiomer.

A convenient starting material useful for obtaining the desired compounds $R^2R^3N$-(B) is the correspondingly substituted 8-hydroxybenzazepine having the symbolic formula HO-(B) . Thus, an 8-hydroxy-substituted benzazepine may be treated with acetone and chloroform in the presence of base [see for example, Synthesis, 31(1977)] to produce a carboxylate salt having the formula

$$M^{+-}O_2C-C(CH_3)_2-O-\text{(B)}$$

wherein $M^+$ represents one equivalent of a metal cation such as a sodium or a potassium cation which is derived from the particular base that was used in the reaction. Use of a base comprising a multivalent metal such as calcium will produce a corresponding salt, e.g. $Ca(O_2C-C(CH_3)_2-O-\text{(B)})_2$. The so-produced carboxylate salt may next be converted into the corresponding acid halide such as the acid chloride by treatment with for example thionyl chloride. The so-produced acid halide may next be converted into the corresponding acid amide by treatment with concentrated ammonium hydroxide in a suitable solvent such as tetrahydrofuran. The so-produced acid amide having the formula

$$H_2NCO-C(CH_3)_2-O-\text{(B)}$$

may next be converted into the acylanilide having the formula $HOC(CH_3)_2CONH-\text{(B)}$ by treatment with a strong base in a suitable solvent, e.g. sodium hydride in N,N-dimethylformamide [see for example Organic Reactions, 18, 99 (1970)]. The so-obtained amide may then be converted to the corresponding amine, compound I wherein $R^2=R^3=H$,

$H_2N$-(B)

by hydrolysis, especially with an acid, in particular mineral acid, for example with 50% sulfuric acid solution.

If desired, the so-obtained amine may be subjected to a number of finishing steps selected from steps (a) to (p) given above.

For example, the elimination of a 7-halogen atom (chloro, bromo or iodo) can be effected by means of hydrogen and a catalyst, e.g. 20% palladium on charcoal, in an inert organic solvent such as a lower alkanol, e.g. ethanol or methanol.

The oxidative condensation of an arylmethylthioether with a 7-unsubstituted-8-$NH_2$ benzazepine can be effected using N-chlorosuccinimide or especially _t_-butyl hypochlorite as oxidising agent. The aryl group in the arylalkylthioether is preferably a substituted or unsubstituted phenyl group; a preferred ether is thioanisole. The reaction is preferably carried out at reduced temperature, e.g. at below 0°C., preferably at about -30°C., in an inert organic solvent which may comprise a haloalkane, e.g. a methylene chloride:acetonitrile mixture. After basification (e.g. with sodium in methanol) and evaporation to dryness, the residue is heated in a basic medium, e.g. a tertiary organic base in an inert hydrocarbon solvent such as toluene. The resulting 7-arylthioalkyl-benzazepine can then be reduced to the 7-alkyl-benzazepine, e.g. with lithium aluminum hydride in an ethereal solvent or with catalytic reduction, e.g. with palladium on carbon or with Raney nickel.

A so-obtained amine $H_2N$-(B) may be reacted with an acylating agent such as $ClSO_2R^a$, $R^aSO_2NCO$, $R^aNCO$,

$R^aCOCl$, $(R^aCO)_2O$, $R^aXCOCl$ or $(R^aSO_2)_2O$ to produce the corresponding sulfonamide, sulfonylurea, acyl amide, urethane, or urea (wherein $R^a$ and X are as defined above). This reaction is preferably carried out in the presence of an organic tertiary amine base such as triethylamine, N-methyl morpholine or pyridine, or a weak inorganic base such as sodium bicarbonate or potassium carbonate.

Alternatively, compounds of the formula

$$R^aX.CO.NH\text{-}(B)$$

wherein X is O or $NR^b$ may be prepared by reaction of $H_2N$-(B) with carbonyldiimidazole, followed by the _in situ_ reaction of the intermediate urea with a primary or secondary alcohol or a primary or secondary amine. Reaction with ammonia will produce compounds wherein $R^aX$ is $NH_2$.

The acylamido derivatives having the formula $R^aCONH$-(B) wherein $R^a$ is as defined herein may be reduced with for example lithium aluminum hydride or diborane to produce a substituted amine

$$R^xNH\text{-}(B)$$

wherein $R^x$ may be chosen from those groups as defined herein for $R^a$, provided that _m_ is not 0.

Reduction of urethanes of the formula

$$R^aO.CO.NH\text{-}(B)$$

with reducing agents such as lithium aluminum hydride produces compounds of the formula $CH_3NH$-(B) .

If desired the so-obtained amine $R^xNH$-(B) may be acylated with an appropriate acylating agent as described above for the acylation of $H_2N$-(B) to produce the corresponding urethane or acylamido derivatives.

The amides having the formula $R^aSO_2NH$-(B) or $R^aCONH$-(B) , may be alkylated by known procedures for

example with an alkyl halide, such as $R^xBr$, wherein $R^x$ is defined herein, in the presence of sodium hydride to produce a disubstituted amide having the structure $R^aCO.R^xN$-(B) or $R^aSO_2.R^xN$-(B) .

These amides may be reduced with for example lithium aluminum hydride to produce the corresponding tertiary amine (from the carboxamide) or secondary amine $R^xNH$-(B) (from the sulfonamide). Alternatively, the carboxamide may be hydrolysed e.g. with a base or with acid to remove the acyl group $R^aCO$ and thereby produce the corresponding secondary amine $R^xNH$-(B) .

The so-produced acylamido derivatives having the formula $(R^aCO)R^xN$-(B) (wherein $R^a$ and $R^x$ may be the same or different and are as defined herein) may be reduced with, for example lithium aluminum hydride or diborane to produce a disubstituted amine

$R^aR^xN$-(B)

wherein $R^a$ and $R^x$ may be the same or different and represent certain $R^2$ and $R^3$ substituents as defined herein. Reduction of urethanes of the formula $R^aO.CO.N(R^x)$-(B) with reducing agents such as lithium aluminum hydride produces compounds of the formula $R^xCH_3N$-(B).

In the above-described conversion sequences, the proper selection of the acylating agent (e.g. $R^aCOCl$) and alkylating agent (e.g. $R^xBr$) will permit the preparation of the desired compounds of formula I, i.e.

$R^2R^3N$-(B)

Other methods for obtaining compounds of formula I,

$R^2R^3N$-(B)

from compounds $R^3NH$-(B) , wherein $R^2$ is $R^a$ as defined above and $R^3$ is $R^a$ as defined above or a hydrogen atom,

are well known in the art. Thus, for example, compounds $R^3NH$-Ⓑ can be reductively alkylated with a suitable aldehyde or ketone such as $R^aCHO$ (wherein $R^a$ is as defined herein), in the presence of a reducing agent such as $NaCNBH_3$ for example, in a suitable solvent such as methanol, to produce an amine having the structure

$R^xR^3N$-Ⓑ

wherein $R^x$ and $R^3$ may be the same or different and are as defined herein.

When $R^3$ is hydrogen, this procedure may be repeated to produce compounds

$R^xR^3N$-Ⓑ

wherein $R^3$ is no longer a hydrogen atom and $R^x$ and $R^3$, which may be the same or different, are otherwise as defined herein.

Compounds having structural formula I of the type

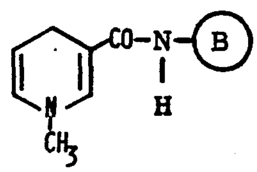

may be prepared from compounds of formula I of the type

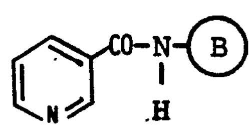

by N-methylation of the pyridine ring, for example with methyl iodide, followed by 1,4-reduction of the resulting N-methyl pyridinium salt, for example with sodium dithionite in a suitable solvent such as aqueous ethanol.

In order to obtain products of the invention of formula I wherein $R^2$ is the group Aryl as hereinbefore defined, one may use the well-known Chapman rearrangement [Org. Reactions, 14, 1 (1965)] as exemplified below:

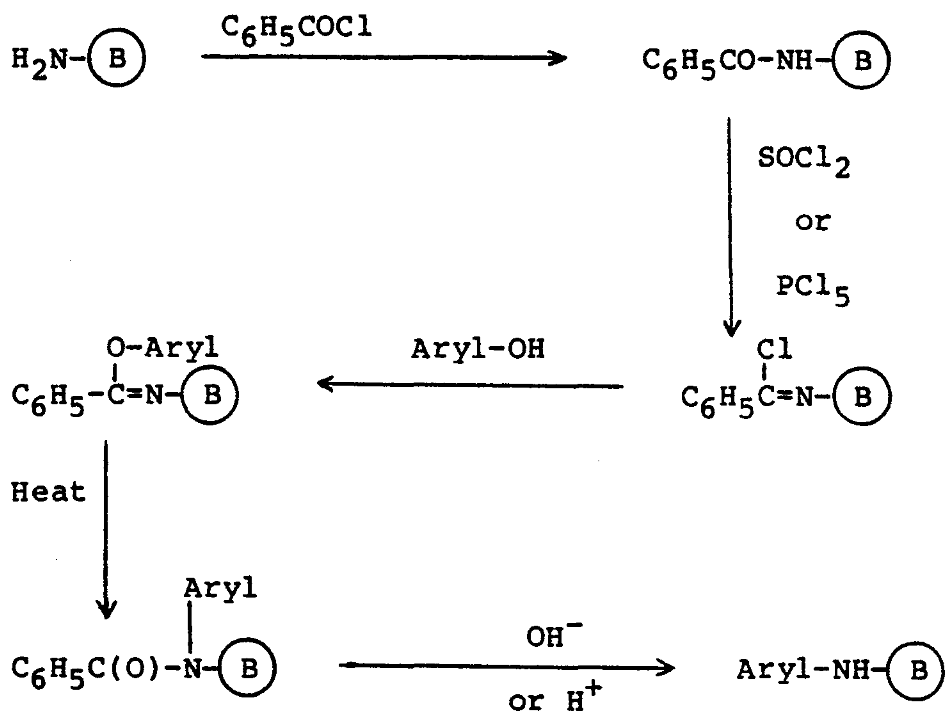


(wherein the group Aryl is as hereinbefore defined).

Alternatively, the Goldberg diarylamine synthesis [Berichte, 40, 4541 (1907)] using N-acetyl derivatives may be employed:

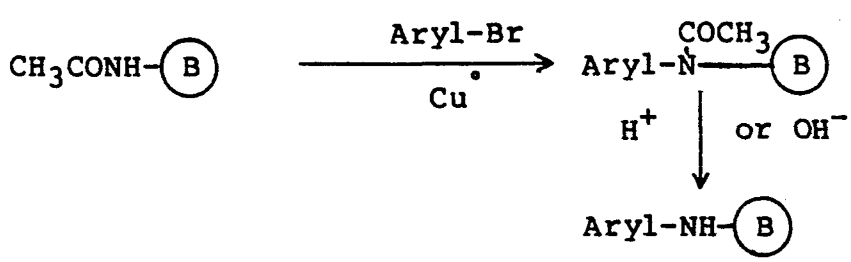

wherein the group Aryl is as hereinbefore defined.

A compound having structural formula I, wherein $R^2$ and $R^3$ taken together with the nitrogen atom to which they are attached form a ring, may be prepared from the corresponding compound having structural formula I wherein $R^2$ and $R^3$ are both hydrogen. For example, the primary amino compound may be reacted with an acylating agent such as an acid chloride having the structural formula $L(CH_2)_nCH_2-YCH_2COCl$ wherein L is a leaving group such as halogen and Y and n are as defined herein to produce the amides having the structural formula

$$L(CH_2)_nCH_2YCH_2CONH-(B)$$

wherein L, Y and n are as defined herein.

The amide may be reduced to the corresponding amine, for example with diborane, and cyclized by heating in the presence of an acid acceptor such as pyridine to produce the cyclic amine having the following structural formula

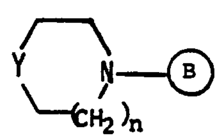

The amides may also be cyclized, for example by use of sodium hydride, to produce the corresponding cyclic amides having the structural formula

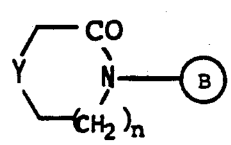

which may also be reduced, for example with lithium aluminum hydride, in an inert organic solvent, to produce the same cyclic amine described above.

In order to obtain products of this invention having structural formula I wherein $R^5$ and/or $R^6$ is hydroxy, it will be clear to those skilled in the art that the oxygen substituent or substituents should be suitably protected during many of the above-described chemical transformations. A convenient protecting group is for example the benzyl group, which can be removed by hydrogenolysis.

The compounds of formula I can be isolated as their *R* or *S* enantiomers, especially if the starting material $HO.C(CH_3).CO.NH$-Ⓑ has the desired *R* or *S* configuration. Alternatively, they can be isolated as their racemates which, if desired, can be resolved by standard methods into the *R* and *S* enantiomers.

The 8-hydroxy-substituted benzazepine compounds HO-Ⓑ may be prepared for example by methods described in U.S. Patent 3,393,192 or by variations thereof known in the art.

The compounds of the invention display pharmacological activity in test procedures designed to show antipsychotic and antidepressant activity.

Preferred compounds of the invention include:
(R)-8-dimethylamino-3,7-dimethyl-1-phenyl-2,3,4,5-tetrahydro-1*H*-3-benzazepine,
(R)-3,7-dimethyl-8-ethylamino-1-phenyl-2,3,4,5-tetrahydro-1*H*-3-benzazepine,
(R)-7-chloro-8-dimethylamino-3-methyl-1-phenyl-2,3,4,5-tetrahydro-1*H*-3-benzazepine, m.p. (maleate) 155-157°C.,
(R)-3,7-dimethyl-8-formamido-1-phenyl-2,3,4,5-tetrahydro-1*H*-3-benzazepine,

(R)-7-chloro-8-ethylamino-3-methyl-1-phenyl-2,3,4,5-tetrahydro-1H-3-benzazepine, m.p. 120-121°C., (R)-7-chloro-8-formamido-3-methyl-1-phenyl-2,3,4,5-tetrahydro-1H-3-benzazepine, m.p. 135-136°C., (R)-7-chloro-3-methyl-8-methylamino-1-phenyl-2,3,4,5-tetrahydro-1H-3-benzazepine, m.p. 98-100°C., (R)-3,7-dimethyl-8-methylamino-1-phenyl-2,3,4,5-tetrahydro-1H-3-benzazepine, (R)-3,7-dimethyl-8-methanesulfonamido-1-phenyl-2,3,4,5-tetrahydro-1H-3-benzazepine, m.p. 113-115°C., and the compounds of the Examples.

## ANTIPSYCHOTIC POTENTIAL

### BLOCKADE OF AMPHETAMINE-INDUCED LETHALITY IN AGGREGATED MICE

Amphetamines are known to be more lethal in grouped than singly-housed mice [Psychopharmacologia, 1, 210 (1960)]. Standard antipsychotic drugs are potent antagonists of this aggregate toxicity [Arch. Int. Pharmacodyn, 113, 290 (1958)] and the ability of drugs to antagonize amphetamine-induced lethality is used to predict antipsychotic potency [Arzneim.-Forsch., 25, 1436 (1975)].

### METHODS AND MATERIAL

Methamphetamine was used to produce lethality in groups of ten mice housed in 11 x 26 x 13 $cm.^3$ plastic chambers. Test drugs were administered 30 minutes prior to intraperitoneal injection of methamphetamine at 15 mg/kg, a dose that typically killed at least 90% of the mice within 4 hours. The number of deaths in each group was recorded 4 hours after methamphetamine administration. The dose of each test compound that provided 50% protection ($ED_{50}$) and the 95% confidence limits (95% CL) were determined using probit analysis.

### RESULTS

A representative compound of the invention, (R)-8-amino-7-chloro-3-methyl-1-phenyl-2,3,4,5-tetrahydro-1H-3-benzazepine, caused dose-related blockade of methamphetamine-induced lethality of grouped mice and had an $ED_{50}$=13.5 mg/kg when given by the oral route.

Representative compounds of the invention also displayed activity when tested as follows:

CONDITIONED AVOIDANCE SUPPRESSION IN RATS

Clinically active antipsychotic drugs are known to depress discrete trial avoidance behavior at doses that do not retard escape responding [Ann. N.Y. Acad. Sci., 66, 740 (1957)]. A series of experiments was carried out to assess the ability of the compounds of the invention to suppress the conditioned avoidance response (CAR) in rats.

METHODS AND MATERIALS

Rats were required to jump onto a platform located 17.15 cm. (6-3/4 inches) above the grid floor of an experimental chamber in response to a 5-second tone to avoid a ten-second foot shock (0.6 MA). Each experimental session consisted of 20 such trials presented at 30-second intervals. A correct CAR occurred whenever the rat jumped onto the platform during the tone (prior to foot shock). An escape response occurred when the rat jumped onto the platform during shock. A response failure is defined as the lack of an escape response during the 10-second shock period.

Groups of 6-8 rats were trained on two consecutive days (total of 40 trials). Rats that reached criterion on day 2 (correct CARs on 16 or more of the 20 trials) were treated with either a test drug or a vehicle on day 3. Suppression of CAR was analyzed statistically using Student's t-test comparing the performance of drug-treated to vehicle-treated rats. The minimal effective dose (MED) for each drug is defined as the lowest dose tested that significantly ($P<.05$) reduced avoidance responding.

RESULTS

A representative compound of the invention, (R)-8-amino-7-chloro-3-methyl-1-phenyl-2,3,4,5-tetrahydro-1H-3-benzazepine, compound A, when tested by the above procedure, manifested a dose-related specific blockade of conditioned avoidance responding with an MED of 2.5 mg/kg (orally) at 1 and 4 hours after dosing. In this test, (R)-(+)-7-chloro-8-methanesulfonamido-3-methyl-1-phenyl-1,2,3,4-tetrahydro-1H-3-benzazepine (as maleate), compound B, and (R)-(+)-7-chloro-3-methyl-8-methylamino-1-phenyl-2,3,4,5-tetrahydro-1H-3-benzazepine, compound C, had respective MEDs of 3 and 10 mg/kg (orally) at 1 hour after dosing.

BINDING TO D-1 RECEPTORS

The foregoing tests indicate that compounds of formula I have potential as anti-psychotic drugs. Such drugs usually act at D2 receptors, but are less active or not active at all at D1 receptors. Surprisingly, we have found that compounds of formula I act much more strongly at D1 receptors than at D2 receptors, as indicated by the following Table:

| Test Compound | $K_i$ (nM) Displacement of Reference Compound | |
|---|---|---|
| | 1 | 2 |
| A | 2.1 | 1,013 |
| B | 29 | 10,430 |
| C | 11 | 1,035 |

Test compounds A, B and C as identified above.

Reference compounds (both tritium-labelled):

1 is (+)-7-chloro-8-hydroxy-3-methyl-1-phenyl-2,3,4,5-tetrahydro-1H-3-benzazapine (as maleate), which acts at D1 receptors; 2 is spiperone, a known anti-psychotic which acts at D2 receptors.

The lower the figure in columns designated 1 and 2, the higher the activity of the test compound A, B or C; the figure gives the concentration (nM) of test compound at the D1 or D2 receptors needed to displace 50% of the bound reference compounds 1 and 2 from their respective receptor sites. Hence the test compounds of formula I have a different profile of anti-psychotic effect from known anti-psychotics such as spiperone, and can thus be expected to lack many side-effects associated with those compounds.

## ANTIDEPRESSANT POTENTIAL

### 1. EFFECTS ON TETRABENAZINE-INDUCED PTOSIS IN MICE

Clinically active antidepressant drugs are known to block tetrabenazine-induced ptosis in mice (Psychosomatic Medicine, Nodine and Moyer, Eds., Lea and Febiger, Philadelphia, 1962, pp 683-90). Activity in this test is used to predict anti-depressant activity in man.

### METHODS AND MATERIALS

Groups of 5 mice were administered test drugs followed 30 minutes later by ip injection of tetrabenazine (TBZ), 30 mg/kg. Thirty minutes later, the degree of ptosis was evaluated. Percent blockade of each treated group was used to determine $ED_{50}$'s, defined as that dose which prevents ptosis in 50% of mice. $ED_{50}$'s and 95% confidence limits were calculated by probit analysis.

RESULTS

(R)-8-Amino-7-chloro-3-methyl-1-phenyl-2,3,4,5-tetrahydro-1H-3-benzazepine, a representative compound of the invention, caused antagonism of TBZ-induced ptosis in mice at an $ED_{50}$ of 3 mg/kg when administered orally.

2. EFFECTS ON MURICIDE ACTIVITY IN RATS

Blockade of muricide (mouse-killing) activity in rats has been used as a measure of evaluating the anti-depressant activity of drugs (Int. J. Neuro-pharmacol., 5, 405-11 (1966)).

METHODS AND MATERIALS

Groups of 5 rats were administered test drug intraperitoneally and tested 30 and 60 minutes later for presence of muricide activity. Percent blockade of each treated group using data obtained at both these time points was calculated and dose-response data were used to determine each $ED_{50}$. $ED_{50}$ is defined as that dose which blocks muricide behavior in 50% of treated rats and was calculated using probit analysis.

RESULTS

A representative compound of this invention, (R)-8-amino-7-chloro-3-methyl-1-phenyl-2,3,4,5-tetrahydro-1H-3-benzazepine, blocked muricide behavior at an $ED_{50}$ of 2.7 mg/kg.

Pharmaceutical activity of the type described above is most pronounced for those compounds of the invention wherein the stereochemical configuration at the asymmetric carbon atom, i.e. carbon number 1 in structural formula I, is R.

The compounds of the invention form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic, and the like. The salts are prepared by contacting the free base form with a sufficient amount of the desired acid to produce a salt in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention.

For preparing pharmaceutical compositions from the compounds described by this invention, inert, pharmaceutically acceptable carriers can be either solid or liquid. Solid form preparations include powders, tablets, dispersible granules, capsules, cachets and suppositories. A solid carrier can comprise one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In tablets the active compound is mixed with carrier having the necessary binding properties in suitable proportions and compacted

into the shape and size desired. The powders and tablets preferably contain from 5 to about 70 percent, preferably from 10 to about 70 percent, of the active ingredient. Suitable solid carriers include magnesium carbonate, magnesium stearate, talc, sugar, lactose, pectin, dextrin, starch, gelatin, tragacanth, methylcellulose, sodium carboxymethylcellulose, low melting waxes and cocoa butter. The active compound can also be formulated with encapsulating material as carrier to provide a capsule or cachet in which the active component (with or without other carriers) is surrounded by carrier, which is thus in association with it. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As examples there may be mentioned water or water-propylene glycol solutions for parenteral injection. Liquid preparations can also be formulated in solution in aqueous polyethylene glycol solution. Aqueous solutions suitable for oral use can be prepared by adding the active ingredient to water and adding suitable colorants, flavors, stabilizing, sweetening, solubilizing and thickening agents as desired. Aqueous suspensions suitable for oral use can be made by dispersing the finely divided active ingredient in water with viscous material, i.e., natural or synthetic gums, resins, methylcellulose, sodium

carboxymethylcellulose and other well-known suspending agents.

Also included are solid form preparations which are intended to be converted, shortly before use, into liquid form preparations for either oral or parenteral administration. Such liquid forms include solutions, suspensions and emulsions. These particular solid form preparations are most conveniently provided in unit dose form and as such are used to provide a single liquid dosage unit. Alternately, sufficient solid may be provided so that after conversion into liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended for conversion to liquid form may contain, in addition to the active material, flavors, colorants, stabilizers, buffers, artificial and natural sweeteners, dispersants, thickeners, solubilizing agents and the like. The solvent used for preparing the liquid form preparation may be water, isotonic water, ethanol, glycerine, propylene glycol and the like as well as mixtures thereof. Naturally, the solvent used will be chosen with regard to the route of administration; for example, liquid preparations containing large amounts of ethanol are not suitable for parenteral administration.

The invention also contemplates mechanical delivery systems, e.g. for transdermal delivery.

Preferably, the pharmaceutical preparation is

in unit dosage form, each dose containing an appropriate quantity of the active component. The unit dosage form can be a packaged preparation, the package containing discrete quantities of preparation, for example, packaged tablets or capsules, or powders in vials or ampoules. The unit dosage form can also be a capsule, cachet or tablet itself or it can be the appropriate number of any of these in packaged form.

The quantity of active ingredient in a unit dose of preparation may be varied or adjusted from 1 mg to 100 mg. according to the particular application and the potency of the active ingredient. The composition can, if desired, also contain other therapeutic agents.

The dosages may be varied depending upon the requirement of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following Examples illustrate but do not limit the invention:

EXAMPLE I

(R)-8-Amino-7-chloro-3-methyl-1-phenyl-2,3,4,5-tetrahydro-1H-3-benzazepine

A. (R)-1,1-Dimethyl-(7-chloro-3-methyl-1-phenyl-2,3,4,5-tetrahydro-1H-3-benzazepine-8-yl-oxy)acetamide

Sodium hydroxide (20.0g, 0.5 mol) was added to a stirred solution of (R)-7-chloro-8-hydroxy-3-methyl-1-phenyl-2,3,4,5-tetrahydro-1H-3-benzazepine (21.6g, 0.075 mol) in acetone (900 ml) and the mixture was heated to reflux to effect solution. Chloroform (13.4g, 0.112 mol) was added dropwise during one hour and then the mixture was heated under reflux for 5 hours and allowed to cool.

The solid was filtered off, washed with ether and dried. Yield 47.6 g. This material was added with stirring to thionyl chloride (400 ml) at room temperature and stirring continued for 1/2 hour. The excess thionyl chloride was evaporated off below 50° and the residue was dissolved in dry tetrahydrofuran and added to stirred concentrated ammonia. After the addition (1 hr) the mixture was allowed to stand overnight.

The solid was filtered off, washed with water and dried. Yield 17.2g, light brown powder. This was dissolved in chloroform and passed through a filter of silica gel. The filtrate was evaporated and the residue recrystallized from chlorobutane to give 13.2 g of pale lemon colored needles m.p. 151-153°C.
$[\alpha]^{26}_{D}$52.9°(DMF,c=1).

B. (R)-7-Chloro-8-[N-(2-hydroxy-2-methylpropionyl)]amino-3-methyl-1-phenyl-2,3,4,5-tetrahydro-1H-3-benzazepine

The product obtained in Part A (13.2g, 0.0356 mol) was dissolved in anhydrous dimethylformamide (120 ml) and 50% sodium hydride in mineral oil (1.71g, 0.0356 mol) was added. The mixture was heated and stirred to 110° C. under nitrogen for 24 hours.

The mixture was cooled and poured onto ice-water (1200 ml). The precipitated white solid was filtered off and dried *in vacuo*.

Recrystallization from chlorobutane gave 10.3 g of colorless solid, m.p. 174-176°C. $[\alpha]^{26}_{D}$66.6°(DMF,c=1).

C. (R)-8-Amino-7-chloro-3-methyl-1-phenyl-2,3,4,5-tetrahydro-1H-3-benzazepine

The product obtained in Part B (110.0g) was added to 400 ml. of 50% sulfuric acid, and the mixture stirred at 100° for 2 hrs. and then allowed to cool. The solution was then poured into 2 liters of 6N NaOH, and extracted with two 1 liter portions of ethyl acetate. The combined extracts were washed with water (1 liter), dried over anhydrous magnesium sulfate and evaporated *in vacuo*. The resulting oil was recrystallized from ethanol to give 50.1g. of product as a monoethanolate, m.p. 68-70°C, $[\alpha]^{26}_{D}$50.7°(DMF,c=1).

EXAMPLE II

(R)-8-Amino-7-chloro-3-methyl-1-phenyl-2,3,4,5-tetrahydro-1H-3-benzazepine, maleate salt

14.1g. of the free base of Example IC was dissolved in warm ethyl acetate (300 ml.), and maleic acid (4.92 g.) was added. The resulting mixture was brought to reflux, then allowed to cool. The resulting solid was filtered off and recrystallized from ethanol to give colorless crystals of the maleate, m.p. 203-5°C, $[\alpha]^{26}_{D}$-2.9°(DMF,c=1).

EXAMPLE III

(R)-8-Methanesulfonamido-7-chloro-3-methyl-1-phenyl-2,3,4,5-tetrahydro-1H-3-benzazepine, maleate salt

The compound produced in Example IC (1.66g.) was dried *in vacuo* to remove ethanol, and dissolved in 100 ml. of acetonitrile. Sodium bicarbonate (10g.) was added, and then methanesulfonyl chloride (2.5g.) added dropwise to the stirred mixture over 2 hrs. The mixture was then stirred overnight and poured into water, and the resulting mixture extracted with ether. The extracts were dried over anhydrous magnesium sulfate and then evaporated *in vacuo.*

Chromatography of the residue on silica gel using chloroform and grading to ethyl acetate gave material in the ethyl acetate eluates which formed a salt on treatment with maleic acid. Recrystallization from methanol- ethyl acetate gave 1.2g. product m.p. 162-164°C, $[\alpha]^{26}_{D}$2.3°(c=1, DMF).

EXAMPLE IV

(R)-8-Acetamido-7-chloro-3-methyl-1-phenyl-2,3,4,5-tetrahydro-1H-3-benzazepine

A solution of the compound produced in Example IC (5.3 g.) in 100 ml. of acetonitrile was treated with 5 ml. of acetic anhydride and 20 mg. of 4-dimethylaminopyridine. The mixture was stirred at room temperature for 5-1/2 hrs., after which it was evaporated to dryness and 25 ml. of methanol and another 25 mg. of dimethylaminopyridine were added. The mixture was refluxed for 20 min., cooled, and evaporated to dryness. The residue was stirred with 90 ml. 5% sodium carbonate solution, and the solids were filtered off. Recrystallization of this material from acetonitrile gave 4.05 g. product which was dried *in vacuo* at 50°C to give material m.p. 105-110°C, $[\alpha]^{26}_{D}$39° (c=1, DMF).

EXAMPLE V

(R)-8-Amino-3-methyl-1-phenyl-2,3,4,5-tetrahydro-1H-3-benzazepine

(R)-8-Amino-7-chloro-3-methyl-1-phenyl-2,3,4,5-tetrahydro-1H-3-benzazepine (from Example 1) (0.01 mole) in 100 ml. of ethanol was hydrogenated in a Parr apparatus over 400 mg. of 20% $Pd(OH)_2/C$ for twelve and a half hours. The catalyst was filtered off, and the solvent was evaporated off to give a solid residue which was partitioned between dilute aqueous sodium hydroxide and ether. The ether layer was separated and dried over anhydrous potassium carbonate. Evaporation to a volume

of about 20 ml. resulted in crystallization of the product, which was filtered off and dried; 1.35 g, m.p. 145-7°C., $[\alpha]^{26}_D$ +70.6° (c=1, DMF).

## EXAMPLE VI

### (R)-8-Amino-3,7-dimethyl-1-phenyl-2,3,4,5-tetrahydro-1H-3-benzazepine

A. A solution of *t*-butyl hypochlorite (4.75 g.) in 20 ml. of methylene dichloride was added dropwise to a mixture of the 8-amino-benzazepine from Example V (8.65 g., 34 mmol) and thioanisole (8.45 g., 68 mmol) in 300 ml. of 3:1 acetonitrile:methylene chloride at -30°C. The homogeneous solution was stirred at -30°C. for 4 hours, and then a solution of 1.6 g. of sodium in 25 ml. of methanol was added dropwise. The mixture was allowed to come to room temperature over 90 minutes and then evaporated to dryness *in vacuo*. A solution of 25 ml. of triethylamine in 200 ml. of toluene was added to the residue which was then refluxed for seven and a half hours. The mixture was cooled and extracted with two 200 ml. portions of 2N HCl. The extracts were basified with 10% NaOH and extracted with two 100 ml. portions of ether. The ether extracts were dried and evaporated to dryness. The residue was chromatographed on silica gel, eluting with 50:45:5 chloroform:ethyl acetate:ethanol to give a product (1.1 g.) which was carried to the next step without further purification.

B. The preceding material (1.1 g.) was dissolved in 45 ml. of dry ether and treated with 280 mg. of lithium aluminum hydride. After stirring 8 hours at room

temperature, the mixture was hydrolyzed with water and the ether layer was separated, dried and evaporated to give an oil. This material was chromatographed on a silica gel column eluting with 50:45:5 chloroform:ethyl acetate:ethanol to give 250 mg. oily product. Treatment of this product with 80 ml. of maleic acid gave the salt (from isopropanol) m.p. 171-3°, $[\alpha]^{26}_{D}$+7.0°(c=0.25, DMF).

CLAIMS

1. Compounds having the structural formula I

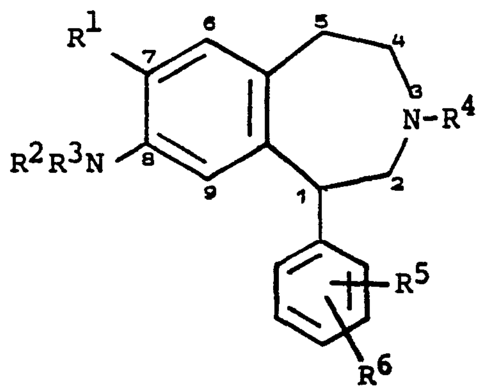

I

wherein $R^1$ is a hydrogen or halogen atom or an alkyl, $S(O)_n$.alkyl or alkoxy group having from 1 to 6 carbon atoms, where <u>n</u> is 0, 1 or 2, or a trifluoromethyl group;

$R^2$ is a hydrogen atom, a group $R^a$ [wherein $R^a$ is an alkyl group having from 1 to 6 carbon atoms, a group $-(CH_2)_m)$-Aryl (wherein <u>m</u> is 0 or an integer of 1 to 3 and Aryl is a group

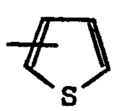, 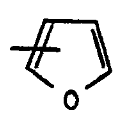, 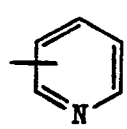, 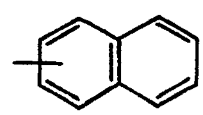 or 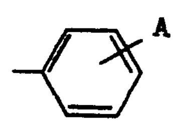

wherein A is a hydrogen or halogen atom, an alkyl, $S(O)_n$.alkyl or alkoxy group having from 1 to 6 carbon atoms, wherein <u>n</u> is 0, 1 or 2, or a phenyl, hydroxy or trifluoromethyl group)], or a group $C(O)XR^a$ [wherein X is a bond, O, or $NR^b$ (wherein $R^b$ is a hydrogen atom or an alkyl group having from 1 to 6 carbon atoms), $R^a$ is as defined above and $XR^a$ may be $NH_2$],

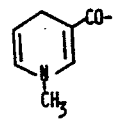,

$SO_2R^a$ or $CONHSO_2R^a$ (wherein $R^a$ is as defined above);

$R^3$ is a hydrogen atom or a formyl group or, when $R^2$ is an alkyl group having from 1 to 6 carbon atoms, $R^3$ can also be an alkyl group having from 1 to 6 carbon atoms or a group $R^a$ as defined above;

or $R^2$ and $R^3$ together with the nitrogen atom to which they are attached form a group

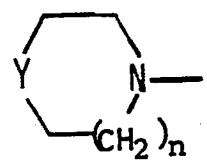

wherein Y is O, $CH_2$ or $NR^b$ [wherein $R^b$ is as defined above], and _n_ is 0, 1 or 2 provided that _n_ is not 0 when Y is O or $NR^b$;

$R^4$ is a hydrogen atoms or an alkyl group having from 1 to 6 carbon atom;

each of $R^5$ and $R^6$, which may be the same or different, is a hydrogen or halogen atom or an alkyl or $S(O)_n$.alkyl group having from 1 to 6 carbon atoms, a trifluoromethyl group or a group $OR^b$ [wherein $R^b$ is as defined above]; or $R^5$ and $R^6$ together may form a group $-OCH_2O-$ substituting adjacent positions in the fused benzene ring;

and the pharmaceutically acceptable salts thereof.

2. Compounds and salts as claimed in claim 1 in the form of their R-enantiomers, wherein

$R^1$ is a halogen atom or a methyl or trifluoromethyl group;

$R^2$ is a hydrogen atom or a methyl or $SO_2R^a$ group, wherein $R^a$ is as defined in claim 1;

$R^3$ is a hydrogen atom;

$R^4$ is a methyl group;

$R^5$ is a hydrogen or halogen atom or a methyl group or a group $OR^b$ [wherein $R^b$ is as defined in claim 1]; and

$R^6$ is a hydrogen atom.

3. A compound as claimed in claim 1 or claim 2 wherein $R^5$ and $R^6$ are hydrogen atoms, $R^1$ is a chlorine atom, a hydrogen atom or a methyl group, $R^4$ is a methyl group and $R^3$ is a hydrogen atom or a lower alkyl group.

4. A compound as claimed in any of claims 1 to 3 wherein $R^2$ is a hydrogen atom, a methyl or ethyl group or a group $SO_2R^a$, where $R^a$ is as defined in claim 1 but is preferably an alkyl group.

5. A compound as claimed in claim 1, namely
(R)-(+)-8-amino-3-methyl-1-phenyl-2,3,4,5-tetrahydro-1H-3-benzazepine,
(R)-(+)-7-chloro-8-methanesulfonamido-3-methyl-1-phenyl-2,3,4,5-tetrahydro-1H-3-benzazepine,
(R)-8-acetamido-7-chloro-3-methyl-1-phenyl-2,3,4,5-tetrahydro-1H-3-benzazepine,
(R)-8-amino-3-methyl-1-phenyl-2,3,4,5-tetrahydro-1H-3-benzazepine,
(R)-3,7-dimethyl-8-ethylamino-7-chloro-3-methyl-1-phenyl-2,3,4,5-tetrahydro-1H-3-benzazepine,

(R)-7-chloro-8-dimethylamino-3-methyl-1-phenyl-2,3,4,5-tetrahydro-1H-3-benzazepine,
(R)-3,7-dimethyl-8-formamido-1-phenyl-2,3,4,5-tetrahydro-1H-3-benzazepine,
(R)-7-chloro-8-ethylamino-3-methyl-1-phenyl-2,3,4,5-tetrahydro-1H-3-benzazepine,
(R)-7-chloro-8-formamido-3-methyl-1-phenyl-2,3,4,5-tetrahydro-1H-3-benzazepine,
(R)-7-chloro-3-methyl-8-methylamino-1-phenyl-2,3,4,5-tetrahydro-1H-3-benzazepine,
(R)-3,7-dimethyl-8-methylamino-1-phenyl-2,3,4,5-tetrahydro-1H-3-benzazepine,
(R)-8-dimethylamino-3,7-dimethyl-1-phenyl-2,3,4,5-tetrahydro-1H-3-benzazepine,
(R)-3,7-dimethyl-8-methanesulfonamido-1-phenyl-2,3,4,5-tetrahydro-1H-3-benzazepine, or
(R)-(+)-8-amino-3,7-dimethyl-1-phenyl-2,3,4,5-tetrahydro-1H-3-benzazepine.

6. Pharmaceutically acceptable acid addition salts of the compounds of formula I defined in any of claims 1 to 5.

7. Salts as claimed in claim 6 formed with hydrochloric, sulfuric, phosphoric, acetic, citric, oxalic, malonic, salicylic, malic, fumaric, succinic, ascorbic, maleic or methanesulfonic acid.

8. A process for the preparation of a compound of the formula I defined in claim 1 or of a pharmaceutically acceptable salt thereof, which comprises, as main step, hydrolysing an amide of the formula

$$HO.C(CH_3)_2.CO.NH-\textcircled{B}$$

to an 8-amino-benzazepine of the formula

$H_2N$-(B)

wherein (B) is a benzazepine-8-yl group of the formula

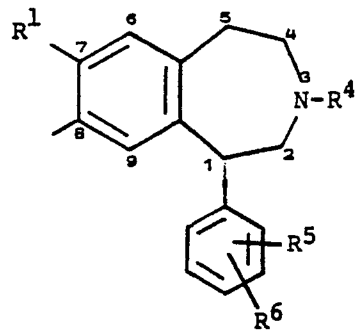

in which $R^1$, $R^4$, $R^5$ and $R^6$ are as defined in claim 1, with the proviso that any hydroxy group in $R^5$ and/or $R^6$ can be protected,

whereafter the resulting 8-amino-benzazepine of the formula

$H_2N$-(B)

can be subjected, as desired or necessary, to one or more of the following finishing steps in any desired order:

(a) Elimination of a 7-halogen atom other than fluorine by catalytic reduction;

(b) Introduction of a 7-alkyl group by oxidative condensation of a 7-unsubstituted-8-$NH_2$-benzazepine with an arylalkylthio ether, followed by reductive cleavage of the resulting 7-arylthioalkyl-benzazepine;

(c) Acylation to a carboxamide $R^aXCO.NH$-(B) or to a sulfonamide $R^aSO_2NH$-(B), wherein X and $R^a$ are as defined in claim 1;

(d) Reaction with carbonyldiimidazole followed by the _in situ_ reaction of the resulting urea with a primary or secondary alcohol $R^aOH$ or with a primary or secondary amine $R^aNH_2$ or with ammonia, where $R^a$ is as defined in claim 1;

(e) Reduction of an acylamide derivative $R^aCONH$-(B) or of a urethane $R^aO(CO)$-NH-(B), wherein $R^a$ is as defined in claim 1, from step (c) to a secondary amine

$R^xNH$-(B) or $CH_3NH$-(B) respectively, where $R^x$ is a group as defined in claim 1 for $R^a$, except that m is not 0;

(f) Alkylation of an amide $R^aSO_2NH$-(B) or $R^aCONH$-(B) from step (c) to yield a disubstituted amide $R^aCO.NR^x$-(B) or $R^aSO_2.NR^x$-(B), where $R^a$ is as defined in claim 1 and $R^x$ is as defined above;

(g) Acylation of a secondary amine $R^xNH$-(B) from step (e) to a carboxamide $R^aX.CO.NR^x$-(B) or to a sulfonamide $R^aSO_2NR^x$-(B), wherein X and $R^a$ are as defined in claim 1 and $R^x$ is as defined above;

(h) Reduction of a carboxamide $R^aCON.R^x$-(B) or $R^aO.CO.NR^x$-(B) from step (f) or (g) to a tertiary amine $R^xR^xN$-(B) or $CH_3N.R^x$-(B) or of a sulfonamide $R^aSO_2.NR^x$-(B) from step (g) to a secondary amine $R^xNH$-(B), wherein $R^a$ is as defined in claim 1 and $R^x$ is as defined above;

(i) Hydrolysis of an amide $R^aCO.NR^x$-(B) from step (f) to a secondary amine $R^x.NH$-(B), wherein $R^a$ is as defined in claim 1 and $R^x$ is as defined above;

(j) Reductive alkylation of the primary amine $H_2N$-(B) from the main step, step (a) or step (b) or of a secondary amine $R^xNH$-(B) from step (e) or step (h) with an aldehyde or ketone and a reducing agent to yield a secondary amine $R^xNH$-(B) or tertiary amine $R^xR^xN$-(B);

(k) Acylation of the primary amine $H_2N$-(B) from the main step, step (a) or step (b), with nicotinic acid or with a reactive derivative thereof to yield an amide of the formula

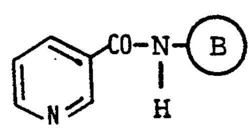

followed by N-methylation in the pyridine ring and 1,4-reduction of the resulting N-methyl-pyridinium salt; (l) Acylation of an amine $H_2N$-(B) with an aromatic carboxylic acid, conversion of the resulting amide _via_ the chloro-imidate into an aryl-imidate by reaction with a phenol of the formula

Aryl-OH,

thermal rearrangement of the aryl-imidate to an aroyl-diarylamine, and hydrolysis of this aroyl-diarylamine to an amine of the formula

Aryl-NH-(B),

wherein Aryl is as defined in claim 1; (m) Condensation of an N-acetyl-amine of the formula $CH_3CONH$-(B) from step (c) with an aryl bromide Aryl.Br in the presence of copper powder to an acetyl-diarylamine, and hydrolysis of this acetyl-diarylamine to an amine of the formula Aryl.NH-(B) wherein the group Aryl is as defined in claim 1; (n) Acylation of the amino compound $H_2N$-(B) with an acylating derivative of the acid $L(CH_2)_nCH_2YCH_2CO_2H$, to produce an amide having the formula

$L(CH_2)_nCH_2YCH_2CONH$-(B)     V

wherein L is a leaving group and Y and _n_ are as defined in claim 1, which is then cyclised to yield a cyclic amide which in turn is reduced to an amine of the formula

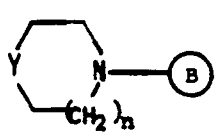

VI

wherein Y and _n_ are as defined in claim 1;

(o) Removal of any group protecting a hydroxy group in $R^5$ and/or $R^6$;

(p) Isolation of the compound of formula I as the free base or as a pharmaceutically acceptable salt, as the racemate or as the resolved R or S enantiomer.

9. A process as claimed in claim 8 wherein the acylation in step (c) or (g) is effected by means of a sulfonyl chloride or anhydride $ClSO_2R^a$ or $(R^aSO_2)_2O$ or an acyl chloride or anhydride $R^aCOCl$, $R^aXCOCl$ or $(R^aCO)_2O$, in the presence of an organic tertiary amine base or a weak inorganic base.

10. Pharmaceutical compositions useful for treating psychoses and/or depression in a mammal which comprise as active ingredient a compound having structural formula I defined in claim 1 or a pharmaceutically acceptable salt thereof in admixture or association with a pharmaceutically acceptable carrier.

11. Compositions as claimed in claim 10 in the form of dosage units preferably containing from 1 to 100 mg. of active ingredient.

12. A method for treating mental disorders such as psychoses and/or depression in a mammal which comprises administering a compound having the formula I defined in claim 1 or a pharmaceutically acceptable salt thereof to said mammal.

European Patent Office

# EUROPEAN SEARCH REPORT

0135767 Application number

EP 84 10 9567

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 033 632 (SMITHKLINE)<br>* Examples 1-6,9-12; claims; page 1, lines 19-25 *<br>--- | 1-4,6-9,10-12 | C 07 D 223/16<br>A 61 K 31/55<br>C 07 D 401/06<br>C 07 D 401/04<br>C 07 D 405/06<br>C 07 D 405/04<br>C 07 D 409/06<br>C 07 D 409/04<br>C 07 D 403/04<br>C 07 D 413/04 |
| Y,D | GB-A-1 268 243 (WALLACE & TIERNAN)<br>* Pages 1-4,34-38; claims *<br>--- | 1-4,6-7,10-12 | |
| A | EP-A-0 005 299 (SCHERICO)<br>* Whole document *<br>--- | 1-4,6-8,10-12 | |
| A | US-A-4 261 890 (WARNER LAMBERT)<br>* Whole document *<br>--- | 1-4,6-7,10-12 | TECHNICAL FIELDS SEARCHED (Int. Cl.4) |
| A | GB-A-1 600 748 (SANDOZ)<br>* Whole document *<br>----- | 1-4,6-7,10-12 | C 07 D 223/00<br>A 61 K 31/00<br>C 07 D 401/00<br>C 07 D 405/00<br>C 07 D 409/00 |

The present search report has been drawn up for all claims

| Place of search | Date of completion of the search | Examiner |
|---|---|---|
| THE HAGUE | 19-11-1984 | NUYTS A.M.K.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document